# FASCICULE DE BREVET EUROPEEN

(11) **EP 1 584 327 B1**
(45) Date de publication et mention de la délivrance du brevet: **28.02.2018**
(21) Numéro de dépôt: 05300247.3
(22) Date de dépôt: 01.04.2005
(51) Int. Cl.: A61K 8/46, A61Q 5/04, A61K 8/67, A61K 8/34

(54) **Procédé de traitement capillaire et utilisation dudit procédé**
Haarbehandlungsverfahren und dessen Einsatz
Hair treatment method and its use

(30) Priorité: 02.04.2004 FR 0450668
(43) Date de publication de la demande: 12.10.2005
(73) Titulaire: L'Oréal, 75008 Paris (FR)
(72) Inventeur: Fondin, Thomas, 95150 Taverny (FR); Sabbagh, Anne, 92500 Rueil Malmaison (FR)
(74) Mandataire: Caillet, Isabelle

(56) Documents cités:
- EP-A- 0 681 828
- US-A- 6 013 249
- DATABASE CAPLUS [Online] CHEMICAL ABSTRACTS SERVICE, COLUMBUS, OHIO, US; XP002307936 extrait de STN Database accession no. 2002:944675 & JP 2002 356408 A (MILBON K.K.) 13 décembre 2002 (2002-12-13)
- DATABASE WPI Section Ch, Week 200107 Derwent Publications Ltd., London, GB; Class D21, AN 2001-052903 XP002340138 -& JP 2000 256146 A (MIRUBON KK) 19 septembre 2000 (2000-09-19) & JP 2000 156146 A 6 juin 2000 (2000-06-06)
- DATABASE WPI Section Ch, Week 200170 Derwent Publications Ltd., London, GB; Class D21, AN 2001-609747 XP002307805 & JP 2001 213741 A (HOYU KK) 7 août 2001 (2001-08-07)
- DATABASE WPI Section Ch, Week 197806 Derwent Publications Ltd., London, GB; Class A25, AN 1978-11057A XP002307937 & JP 52 154529 A (MATSUSHITA ELECTRIC WORKS LTD) 22 décembre 1977 (1977-12-22)

## Description

La présente invention se rapporte à un procédé de traitement des fibres capillaires, ainsi qu'à l'utilisation dudit procédé.

Il est usuel, pour obtenir la déformation permanente des cheveux, de réaliser dans un premier temps l'ouverture des liaisons disulfures de la kératine (cystine) à l'aide d'une composition contenant un agent réducteur, puis après avoir de préférence rincé les cheveux, à reconstituer dans un second temps lesdites liaisons disulfures en appliquant sur les cheveux lissés ou préalablement mis sous tension par des moyens adéquats tels que des bigoudis, une composition oxydante encore appelée fixateur de façon à donner à la chevelure la forme recherchée. Cette technique permet indifféremment de réaliser soit l'ondulation des cheveux, soit leur défrisage, leur crêpage ou leur lissage.

Les compositions réductrices utilisables pour la mise en oeuvre de la première étape de ces procédés contiennent généralement des composés thiolés, tels que l'acide thioglycolique, la cystéine, la cystéamine, l'acide thiolactique et le monothioglycolate de glycérol.

Une telle technique ne donne pas entièrement satisfaction. En effet, cette technique est très efficace pour modifier la forme des cheveux, mais aussi très dégradante pour les fibres capillaires.

Il a en outre été proposé d'élever la température des cheveux, entre l'étape de réduction et l'étape de fixation, au moyen d'un fer chauffant.

Ainsi, la demande de brevet JP 2000 256146 décrit un procédé de déformation permanente des cheveux comprenant l'application d'une composition cosmétique contenant 2 à 11% d'agents réducteurs et de 0,2 à 4% de dithiodiglycolate de diammonium. L'application de la composition réductrice est suivie du passage d'un fer chauffant entre 60 et 220°C.

Cependant, une telle technique d'utilisation d'un fer chauffant nécessite en outre une étape de fixation après le passage du fer, ce qui augmente la durée du traitement.

De plus, la forme obtenue est irréversible. Le contraste entre les parties traitées et les racines est alors important au moment de la repousse des cheveux.

Enfin, si le traitement est effectué sur des cheveux colorés, on constate très souvent une forte altération de la couleur due au traitement.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de modifier le comportement de la fibre capillaire en limitant son altération, de maîtriser le volume des cheveux et d'améliorer les performances cosmétiques des cheveux, notamment la douceur, la brillance et le démêlage en respectant mieux la couleur des cheveux teints.

Ledit procédé doit permettre en outre aux cheveux de garder un aspect naturel, ce qui limite l'effet racines, c'est-à-dire le contraste entre les parties traitées et les racines.

Enfin, l'invention a pour but de réduire la durée du traitement des fibres capillaires et d'obtenir des effets durables.

La Demanderesse a trouvé qu'il était possible de remédier aux inconvénients de l'art antérieur et de remplir les objectifs précités, en mettant en oeuvre un procédé de traitement des fibres capillaires sans fixation comprenant une étape d'application sur les fibres capillaires d'une composition réductrice sans céramide contenant au moins un agent réducteur thiolé et au moins un actif cosmétique, et le ou les actifs cosmétiques étant choisis parmi les actifs non polymériques, puis une étape d'élévation de la température des fibres capillaires, au moyen d'un fer chauffant, à une température au moins égale à 60°C, l'élévation de la température étant effectuée avant ou après le rinçage éventuel des fibres capillaires.

Ainsi, l'invention a pour objet un procédé de traitement des fibres capillaires sans fixation comprenant les étapes suivantes :
- application sur les fibres capillaires d'une composition réductrice sans céramide contenant au moins un agent réducteur thiolé et au moins un actif cosmétique, le ou les actifs cosmétiques étant choisis parmi les actifs non polymériques.
- élévation de la température des fibres capillaires, au moyen d'un fer chauffant, à une température au moins égale à 60°C, l'élévation de la température étant effectuée avant ou après le rinçage éventuel des fibres capillaires.

Sans fixation signifie au sens de la présente invention sans application supplémentaire d'une composition contenant un oxydant chimique tel que le peroxyde d'hydrogène ou un bromate.

De préférence, la composition réductrice ne contient pas d'acide dithiodiglycolique ou l'un de ses sels.

Le ou les actifs cosmétiques non polymériques sont choisis parmi les cires, les agents tensioactifs anioniques, cationiques, non ioniques, amphotères ou zwittérioniques, les agents antichute, les agents antipelliculaires, les agents séquestrants, les agents opacifiants, les colorants, les filtres solaires, les vitamines ou provitamines, les acides gras, les alcools gras, les esters, les pigments nacrés, les huiles minérales, végétales ou synthétiques, ainsi que les parfums et les conservateurs, et leurs mélanges.

Comme vitamines ou provitamines utilisables à titre d'actifs cosmétiques dans la composition réductrice, on peut citer les vitamines A, B3 (la niacinamide), B5, C, E, F et la provitamine B5.

Comme filtres solaires utilisables à titre d'actifs cosmétiques, on peut citer les filtres organiques et les filtres minéraux.

Les filtres organiques peuvent être notamment choisis parmi les dérivés cinnamiques, les dérivés du dibenzoylméthane, les dérivés salicyliques, les dérivés du camphre, les dérivés de triazine tels que ceux décrits dans les demandes de brevet US 4,367,390, EP 0 863 145, EP 0 517 104, EP 0 570 838, EP 0 796 851, EP 0 775 698, EP 0 878 469 , EP 0 933 376 et EP 0 893 119, les dérivés de la benzophénone, les dérivés de β,β'-diphénylacrylate, les dérivés de benzimidazole, les dérivés bis-benzoazolyle tels que ceux décrits dans les brevets EP 0 669 323 et US 2,463,264, les dérivés de méthylène bis-(hydroxyphénylbenzotriazole) tels que ceux décrits dans les demandes US 5,237,071, US 5,166,355, GB-2303549, DE-19726184 et EP 0 893 119, les dérivés de l'acide p-aminobenzoïque, les dimères dérivés d'α-alkylstyrène tels que décrits dans la demande de brevet DE-19855649.

Les filtres minéraux peuvent être choisis parmi les pigments ou bien encore les nanopigments (dont la taille moyenne des particules primaires est généralement comprise entre 5 nm et 100 nm, de préférence entre 10 nm et 50 nm) d'oxydes métalliques, enrobés ou non, comme par exemple des nanopigments d'oxyde de titane (amorphe ou cristallisé sous forme rutile et/ou anatase), de fer, de zinc, de zirconium ou de cérium, qui sont tous des agents photoprotecteurs UV bien connus en soi. Des agents d'enrobage classiques sont par ailleurs l'alumine et/ou le stéarate d'aluminium.

De tels nanopigments d'oxydes métalliques, enrobés ou non enrobés, sont en particulier décrits dans les demandes de brevets EP 0 518 772 et EP 0518773.

Comme acides gras utilisables comme actifs dans le procédé selon l'invention, on peut notamment citer les acides carboxyliques en C₈-C₃₀ saturés ou insaturés, linéaires ou ramifiés, tels que l'acide palmitique, l'acide oléique, l'acide linoléique, l'acide myristique, l'acide stéarique, l'acide laurique, et leurs mélanges.

Comme alcools gras utilisables dans la présente invention, on peut notamment citer les alcools en C₈-C₃₀ saturés ou insaturés, linéaires ou ramifiés comme, par exemple, les alcools palmitylique, oléylique, linoléylique, myristylique, stéarylique et laurylique.

Comme agents tensio-actifs anioniques utilisables dans la présente invention, on peut citer les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates, les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 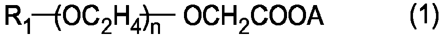 dans laquelle :
R₁ désigne un groupement alkyle alkylamido ou alkaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPOS (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

Comme agents tensio-actifs non-ioniques utilisables dans la présente invention, on peut citer les alcools gras polyéthoxylés, polypropoxylés ou polyglycérolés, les alpha-diols gras polyéthoxylés, polypropoxylés ou polyglycérolés, les alkylphénols gras polyéthoxylés, polypropoxylés ou polyglycérolés et les acides gras polyéthoxylés, polypropoxylés ou polyglycérolés, tous ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine.

Comme agents tensio-actifs amphotères ou zwittérioniques utilisables dans la présente invention, on peut citer les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N⁺(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C) (3)

dans laquelle :
B représente -CH₂CH₂OX', C représente -(CH₂)_{z}-Y', avec z = 1 ou 2, X' désigne le groupement -CH₂CH₂-COOH ou un atome d'hydrogène Y' désigne -COOH ou le radical -CH₂ - CHOH - SO₃H
R₂' désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

Comme agents tensio-actifs cationiques utilisables dans la présente invention, on peut citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Comme huiles végétales utilisables selon la présente invention, on peut notamment citer l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum, et leurs mélanges.

Comme huiles synthétiques utilisables selon la présente invention, on peut citer notamment les polyoléfines en particulier les poly-α-oléfines et plus particulièrement :
- de type polybutène, hydrogéné ou non, et de préférence polyisobutène, hydrogéné ou non.

On utilise de préférence les oligomères d'isobutylène de poids moléculaire inférieur à 1000 et leurs mélange avec des polyisobutylènes de poids moléculaire supérieur à 1000 et de préférence compris entre 1000 et 15000.

A titre d'exemples de poly-α-oléfines utilisables dans le cadre de la présente invention, on peut plus particulièrement mentionner les polyisobutènes vendus sous le nom de PERMETHYL 99 A, 101 A , 102 A , 104 A (n=16) et 106 A (n=38) par la Société PRESPERSE Inc, ou bien encore les produits vendus sous le nom de ARLAMOL HD (n=3) par la Société ICI (n désignant le degré de polymérisation),
- de type polydécène, hydrogéné ou non.

De tels produits sont vendus par exemple sous les dénominations ETHYLFLO par la société ETHYL CORP., et d'ARLAMOL PAO par la société ICI.

Comme esters utilisables selon l'invention, on peut citer notamment le lactate de cétyle ; le lactate d'alkyle en C₁₂-C₁₅ ; le lactate d'isostéaryle ; le lactate de lauryle ; le lactate de linoléyle ; le lactate d'oléyle ; les palmitates d'éthyle et d'isopropyle, les myristates d'alkyle en C₁-C₅ tels que le myristate d'isopropyle, de butyle, le stéarate d'isobutyle ; le malate de dioctyle. On peut aussi citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate, le dicaprate de propylène glycol ; le citrate de triisopropyle ; le citrate de triisotéaryle ; trilactate de glycéryle ; le citrate de trioctyldodécyle ; le citrate de trioléyle.

Comme cires utilisables selon l'invention, on peut citer la cire de Carnauba, la cire de Candelila, l'ozokérite, la cire d'olivier, la cire de riz, la cire de jojoba hydrogénée ou les cires absolues de fleurs telles que la cire essentielle de fleur de cassis vendue par la Société BERTIN (France), la cerabellina ; les cires marines telles que celle vendue par la Société SOPHIM sous la référence M82.

Comme pigments nacrés utilisables selon l'invention, on peut citer le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique.

Parmi les agents antipellicullaires, on peut citer les disulfures de sélénium, les hydroxypyridones et leurs sels tels que l'Octopirex, la pyrithione de zinc et le climbazole.

Parmi les agents antichute, on peut citer le minoxidil et l'aminexil.

Parmi les agents sequestrants, on peut citer l'EDTA, l'acide diéthylènetriamine pentacétique et ses sels.

Parmi les conservateurs, on peut citer l'acide sorbique et ses sels, le phenoxyéthanol, et les sels de l'acide parahydroxybenzoïque

Comme colorants utilisables selon la présente invention, on peut citer les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques utilisables selon l'invention, on peut citer de manière non limitative les composés suivants:
- 1,4-diamino-2-nitrobenzène
- 1-amino-2 nitro-4-β-hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques utilisables selon l'invention on peut citer les colorants azoiques cationiques décrits dans les demandes de brevets WO 95/15144, WO-95/01772 et EP-714954.

Parmi ces composés on peut tout particulièrement citer les colorants suivants:
- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :
- Disperse Red 17
- Acid Yellow 9
- Acid Black 1
- Basic Red 22
- Basic Red 76
- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques on peut citer les colorants suivants :
- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.
Parmi les colorants aziniques on peut citer les composés suivants :
- Basic Blue 17
- Basic Red 2.

Parmi les colorants triarylméthaniques utilisables selon l'invention, on peut citer les composés suivants :
- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Parmi les colorants on peut aussi citer les colorants d'oxydation tels que les bases et les coupleurs.

Parmi les bases d'oxydation, on peut citer les paraphénylènediamines, les paraaminophénols, les orthoaminophénols et les bases hétérocycliques.

Parmi les coupleurs d'oxydation, on peut citer les métadiphénols, les métaaminophénols, métaphénylènediamines, les naphtols et les coupleurs hétérocycliques

Le ou les actifs cosmétiques non polymériques représentent généralement de 0,001 à 30 %, de préférence de 0,1 à 10 %, en poids du poids total de la composition réductrice.

Comme expliqué précédemment, la composition réductrice utilisée dans le procédé selon l'invention comprend un ou plusieurs agents réducteurs choisis parmi les thiols, éventuellement utilisés sous forme de sels.

De préférence, le ou les thiols utilisés comme agents réducteurs dans la composition réductrice sont choisis parmi la cystéïne et ses dérivés, comme la N-acétylcystéïne, la cystéamine et ses dérivés, comme ses dérivés acylés en C₁-C₄ tels que la N-acétyl cystéamine et la N-propionyl cystéamine, l'acide thiolactique et ses esters, comme le monothiolactate de glycérol, l'acide thioglycolique et ses esters, comme le monothioglycolate de glycérol ou de glycol, et le thioglycérol.

Comme thiols utilisables dans la composition réductrice utilisée selon l'invention, on peut encore citer les N-mercapto-alkylamides de sucres tels que le N-(mercapto-2-éthyl)gluconamide, l'acide β-mercaptopropionique et ses dérivés, l'acide thiomalique, la panthétéïne, les N-(mercaptoalkyl)ω-hydroxyalkylamides tels que ceux décrits dans la demande de brevet EP-A-354 835 et les N-mono- ou N,N-dialkylmercapto 4-butyramides tels que ceux décrits dans la demande de brevet EP-A-368 763, les aminomercaptoalkylamides tels que ceux décrits dans la demande de brevet EP-A-432 000 et les alkylaminomercaptoalkylamides tels que ceux décrits dans la demande de brevet EP-A-514 282, le mélange de thioglycolate d'hydroxy-2 propyle (2/3) et de thioglycolate d'hydroxy-2 méthyl-1 éthyle (67/33) décrit dans la demande de brevet FR-A-2 679 448.

Généralement, le ou les thiols utilisés dans la composition réductrice représentent de 0,1 à 30 %, de préférence de 0,5 à 20 %, mieux de 1 à 10 %, en poids du poids total de la composition réductrice.

Selon un mode de réalisation particulièrement préféré, le ou les thiols présents dans la composition réductrice représentent moins de 5% en poids du poids total de la composition réductrice.

Le pH de la composition réductrice est compris généralement entre 2 et 13, de préférence entre 6 et 10, mieux est inférieur ou égal à 9.

Le réglage du pH de la composition peut être obtenu à l'aide d'un agent alcalin, tel que, par exemple, l'ammoniaque ou une amine organique telle que la monoéthanolamine, la diéthanolamine, la triéthanolamine, la 1,3-propanediamine ou le 2-amino-2-méthyl-1-propanol, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

La composition réductrice comprend généralement un ou plusieurs solvants cosmétiquement acceptables, choisis parmi les alcools en C₁-C₆, de préférence les alcanols tels que l'éthanol, le propanol et l'isopropanol, les polyols tels que le propylène glycol, le pentanediol et la glycérine, et les éthers de polyols. La composition réductrice utilisée dans le procédé selon l'invention peut se présenter sous la forme d'une lotion, épaissie ou non, d'une crème, d'un gel ou d'une mousse.

L'application de la composition réductrice telle que définie précédemment constitue donc la première étape du procédé selon l'invention.

De préférence, la composition réductrice est appliquée sur des fibres capillaires humides et propres.

Après l'application de la composition réductrice, on peut laisser poser ladite composition, généralement pendant 5 à 60 minutes, de préférence 5 à 30 minutes, éventuellement sous casque.

Comme expliqué précédemment, le procédé selon l'invention comprend, après l'étape d'application de la composition réductrice une éventuelle étape de rinçage puis une étape d'élévation de la température des fibres capillaires, au moyen d'un fer chauffant, à une température au moins égale à 60°C.

Au sens de la présente invention, on entend par fer un dispositif de chauffage des fibres capillaires par contact.

L'extrémité du fer venant en contact avec les cheveux peut avoir différentes formes. Elle peut notamment présenter une surface plane ; on parle dans ce cas de fer plat. Elle peut également présenter une surface arrondie, on parle dans ce cas de fer rond.

L'application du fer peut se faire par touches séparées successives de quelques secondes, ou par déplacement ou glissement progressif le long des mèches.

A titre d'exemple de fer utilisable dans le procédé selon l'invention, on peut citer tous types de fer plats ou ronds et, en particulier, de manière non limitative, ceux décrits dans les brevets US 4 103 145, US 4 308 878, US 5 983 903, US 5 957 140, US 5 494 058 et US 5 046 516.

De préférence, la température des fibres capillaires est élevée jusqu'à une température comprise entre 60°C et 250°C, mieux entre 120°C et 220°C.

Selon un mode de réalisation préféré, les fibres capillaires ne sont pas rincées avant l'étape d'élévation de la température.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, d'un climazon, ou bien encore par séchage libre.

La présente invention a également pour objet l'utilisation du procédé tel que décrit précédemment pour modifier de manière durable la forme de la chevelure avec une faible dégradation de la couleur des cheveux et/ou une faible dégradation des fibres capillaires.

La présente invention est illustrée par l'exemple suivant.

### Exemple :

On met en oeuvre le procédé de traitement des fibres capillaires selon l'invention en utilisant la composition réductrice suivante :

| | |
|---|---|
| Acide thioglycolique | 1,1 g |
| Niacinamide (vitamine PP ou B3) | 0,1 g |
| 2-amino-2-méthyl-1-propanol | qs pH 9,5 |
| Eau déminéralisée | qsp 100 g |

Les essais sont réalisés sur des cheveux colorés naturellement frisés.

La composition réductrice est appliquée sur la tête. On laisse poser la composition pendant 10 minutes.

On effectue ensuite un pré-séchage partiel des cheveux au moyen d'un sèche-cheveux.

Puis on passe un fer plat chauffé à 180°C.

On constate en final un bon toucher de la fibre, une maîtrise du volume, un bon respect de la couleur et une bonne rémanence des effets dans le temps.

## Revendications

1. Procédé de traitement des fibres capillaires, sans application supplémentaire d'une composition contenant un oxydant chimique, **caractérisé en ce qu'**il comprend les étapes suivantes :
- application sur les fibres capillaires d'une composition réductrice sans céramide contenant au moins un agent réducteur thiolé et au moins un actif cosmétique, le ou les actifs cosmétiques étant choisis parmi les actifs non polymériques choisis parmi les cires, les agents tensioactifs anioniques, cationiques, non ioniques, amphotères ou zwittérioniques, les agents antichute, les agents antipelliculaires, les agents séquestrants, les agents opacifiants, les colorants, les filtres solaires, les vitamines, de préférence les vitamines A, B3, B5 C, E et F, les provitamines, de préférence la provitamine B5, les acides gras, les alcools gras, les esters, les pigments nacrés, les huiles minérales, végétales ou synthétiques, ainsi que les parfums et les conservateurs, et leurs mélanges, la composition réductrice comprenant un ou plusieurs solvants choisis parmi les alcools en C₁-C₆, de préférence les alcanols tels que l'éthanol, le propanol et l'isopropanol, les polyols tels que le propylène glycol, le pentanediol et la glycérine, et les éthers de polyols,
- élévation de la température des fibres capillaires, au moyen d'un fer chauffant, à une température au moins égale à 60 °C, l'élévation de la température étant effectuée avant ou après le rinçage éventuel des fibres capillaires.

2. Procédé selon la revendication 1, **caractérisé en ce que** la composition réductrice ne contient pas d'acide dithiodiglycolique ou l'un de ses sels.

3. Procédé selon l'une quelconque des revendications précédentes, **caractérisé en ce que** le ou les actifs cosmétiques non polymériques représentent de 0,001 à 30 % de préférence de 0,1 à 10 %, en poids du poids total de la composition réductrice.

4. Procédé selon l'une quelconque des revendications précédentes, **caractérisé en ce que** le ou les thiols représentent de 0,1 à 30 %, de préférence de 0,5 à 20 %, mieux de 1 à 10 %, en poids du poids total de la composition réductrice.

5. Procédé selon l'une quelconque des revendications précédentes, **caractérisé en ce que** le ou les thiols représentent moins de 5% en poids du poids total de la composition réductrice.

6. Procédé selon l'une quelconque des revendications précédentes, **caractérisé en ce que** le ou les thiols sont choisis parmi la cystéïne et ses dérivés, comme la N-acétylcystéïne, la cystéamine et ses dérivés, comme ses dérivés acylés en C₁-C₄ tels que la N-acétyl cystéamine et la N-propionyl cystéamine, l'acide thiolactique et ses esters, comme le monothiolactate de glycérol, l'acide thioglycolique et ses esters, comme le monothioglycolate de glycérol ou de glycol, et le thioglycérol.

7. Procédé selon l'une quelconque des revendications précédentes, **caractérisé en ce que** les thiols sont utilisés sous forme de sels.

8. Procédé selon l'une quelconque des revendications précédentes, **caractérisé en ce que** la composition réductrice se présente sous la forme d'une lotion, épaissie ou non, d'une crème, d'un gel ou d'une mousse.

9. Procédé selon l'une quelconque des revendications précédentes, **caractérisé en ce que** la température est élevée jusqu'à une température comprise entre 60 °C et 250 °C, de préférence entre 120 °C et 220 °C.

10. Procédé selon l'une quelconque des revendications précédentes, **caractérisé en ce que** la composition réductrice est appliquée sur des fibres capillaires humides et propres.

11. Procédé selon l'une quelconque des revendications précédentes, **caractérisé en ce que**, après l'application de la composition réductrice, on laisse poser ladite composition réductrice.

12. Procédé selon l'une quelconque des revendications précédentes, **caractérisé en ce que** les fibres capillaires ne sont pas rincées avant l'étape d'élévation de la température.

13. Procédé selon l'une quelconque des revendications précédentes, **caractérisé en ce qu'**il comprend une étape de pré-séchage partiel des fibres capillaires avant l'étape d'élévation de la température.

14. Utilisation du procédé selon l'une quelconque des revendications précédentes, pour modifier de manière durable la forme de la chevelure avec une faible dégradation de la couleur des cheveux et/ou avec une faible dégradation des fibres.

## Patentansprüche

1. Verfahren zur Behandlung von Haarfasern ohne zusätzliche Anwendung einer Zusammensetzung, die ein chemisches Oxidationsmittel enthält, **dadurch gekennzeichnet, dass** es die folgenden Schritte umfasst:
- Aufbringen einer ceramidfreien reduzierenden Zusammensetzung, die mindestens ein Thiol-Reduktionsmittel und mindestens einen kosmetischen Wirkstoff enthält, auf die Haarfasern, wobei der kosmetische Wirkstoff bzw. die kosmetischen Wirkstoffe aus nichtpolymeren Wirkstoffen, die aus Wachsen, anionischen, kationischen, nichtionischen, amphoteren oder zwitterionischen Tensiden, Mitteln zur Bekämpfung von Haarverlust, Antischuppenmitteln, Sequestriermitteln, Trübungsmitteln, Farbmitteln, Lichtschutzmitteln, Vitaminen, vorzugsweise den Vitaminen A, B3, B5, C, E und F, Provitaminen, vorzugsweise Provitamin B5, Fettsäuren, Fettalkoholen, Estern, Perlglanzpigmenten, mineralischen, pflanzlichen oder synthetischen Ölen sowie Duftstoffen und Konservierungsmitteln und Mischungen davon ausgewählt sind, ausgewählt ist bzw. sind, wobei die reduzierende Zusammensetzung ein oder mehrere Lösungsmittel, die aus C₁-C₆-Alkoholen, vorzugsweise Alkanolen wie Ethanol, Propanol und Isopropanol, Polyolen wie Propylenglykol, Pentandiol und Glycerin und Polyolethern ausgewählt sind, umfasst,
- Erhöhen der Temperatur der Haarfasern mit Hilfe eines Heizeisens auf eine Temperatur von mindestens 60°C, wobei die Temperaturerhöhung vor oder nach fakultativem Spülen der Haarfasern durchgeführt wird.

2. Verfahren nach Anspruch 1, **dadurch gekennzeichnet, dass** die reduzierende Zusammensetzung keine Dithiodiglykolsäure oder ein Salz davon enthält.

3. Verfahren nach einem der vorhergehenden Ansprüche, **dadurch gekennzeichnet, dass** der nichtpolymere kosmetische Wirkstoff bzw. die nichtpolymeren kosmetischen Wirkstoffe 0,001 bis 30 Gew.-%, vorzugsweise 0,1 bis 10 Gew.-% des Gesamtgewichts der reduzierenden Zusammensetzung ausmacht bzw. ausmachen.

4. Verfahren nach einem der vorhergehenden Ansprüche, **dadurch gekennzeichnet, dass** das Thiol bzw. die Thiole 0,1 bis 30 Gew.-%, vorzugsweise 0,5 bis 20 Gew.-%, noch besser 1 bis 10 Gew.-% des Gesamtgewichts der reduzierenden Zusammensetzung ausmacht bzw. ausmachen.

5. Verfahren nach einem der vorhergehenden Ansprüche, **dadurch gekennzeichnet, dass** das Thiol bzw. die Thiole weniger als 5 Gew.-% des Gesamtgewichts der reduzierenden Zusammensetzung ausmacht bzw. ausmachen.

6. Verfahren nach einem der vorhergehenden Ansprüche, **dadurch gekennzeichnet, dass** das Thiol bzw. die Thiole aus Cystein und Derivaten davon, wie N-Acetyl-Cystein, Cysteamin und Derivaten davon, wie C₁-C₄-Acylderivaten davon wie N-Acetylcysteamin und N-Propionylcysteamin, Thiomilchsäure und Estern davon, wie Glycerinmonothiolactat, Thioglykolsäure und Estern davon wie Glycerin- oder Glykolmonothioglykolat, Thioglycerin ausgewählt ist bzw. sind.

7. Verfahren nach einem der vorhergehenden Ansprüche, **dadurch gekennzeichnet, dass** die Thiole als Salze eingesetzt werden.

8. Verfahren nach einem der vorhergehenden Ansprüche, **dadurch gekennzeichnet, dass** die reduzierende Zusammensetzung in Form einer verdickten oder unverdickten Lotion, einer Creme, eines Gels oder eines Schaums vorliegt.

9. Verfahren nach einem der vorhergehenden Ansprüche, **dadurch gekennzeichnet, dass** die Temperatur bis zu einer Temperatur zwischen 60°C und 250°C, vorzugsweise zwischen 120°C und 220°C, erhöht wird.

10. Verfahren nach einem der vorhergehenden Ansprüche, **dadurch gekennzeichnet, dass** man die reduzierende Zusammensetzung auf feuchte und saubere Haarfasern aufbringt.

11. Verfahren nach einem der vorhergehenden Ansprüche, **dadurch gekennzeichnet, dass** man nach dem Aufbringen der reduzierenden Zusammensetzung die reduzierende Zusammensetzung einwirken lässt.

12. Verfahren nach einem der vorhergehenden Ansprüche, **dadurch gekennzeichnet, dass** man die Haarfasern vor dem Temperaturerhöhungsschritt nicht spült.

13. Verfahren nach einem der vorhergehenden Ansprüche, **dadurch gekennzeichnet, dass** es einen Schritt der teilweisen Vortrocknung der Haarfasern vor dem Temperaturerhöhungsschritt umfasst.

14. Verwendung des Verfahrens nach einem der vorhergehenden Ansprüche zur dauerhaften Modifizierung der Form des Haars mit geringer Degradation der Farbe der Haare und/oder mit geringer Degradation der Haarfasern.

## Claims

1. Method for treating hair fibres, without additional application of a composition containing a chemical oxidizing agent, **characterized in that** it comprises the following steps:
- applying to the hair fibres a ceramide-free reducing composition containing at least one thiol reducing agent and at least one cosmetic active agent, the cosmetic active agent(s) being chosen from non-polymeric active agents chosen from waxes, anionic, cationic, non-ionic, amphoteric or zwitterionic surfactants, anti-hairloss agents, antidandruff agents, sequestrants, opacifiers, dyes, sunscreens, vitamins, preferably vitamins A, B3, B5, C, E and F, provitamins, preferably provitamin B5, fatty acids, fatty alcohols, esters, pearlescent pigments, mineral, vegetable or synthetic oils, and also fragrances and preservatives, and mixtures thereof, the reducing composition comprising one or more solvents chosen from C₁-C₆ alcohols, preferably alkanols such as ethanol, propanol and isopropanol, polyols such as propylene glycol, pentanediol and glycerol, and polyol ethers,
- increasing the temperature of the hair fibres, by means of a heating iron, to a temperature at least equal to 60°C, the increasing of the temperature being carried out before or after optional rinsing of the hair fibres.

2. Method according to Claim 1, **characterized in that** the reducing composition does not contain dithiodiglycolic acid or a salt thereof.

3. Method according to any one of the preceding claims, **characterized in that** the non-polymeric cosmetic active agent(s) represent(s) from 0.001% to 30%, preferably from 0.1% to 10%, by weight of the total weight of the reducing composition.

4. Method according to any one of the preceding claims, **characterized in that** the thiol(s) represent(s) from 0.1% to 30%, preferably from 0.5% to 20%, better still from 1% to 10%, by weight of the total weight of the reducing composition.

5. Method according to any one of the preceding claims, **characterized in that** the thiol(s) represent(s) less than 5% by weight of the total weight of the reducing composition.

6. Method according to any one of the preceding claims, **characterized in that** the thiol(s) is (are) chosen from cysteine and derivatives thereof, such as N-acetylcysteine, cysteamine and derivatives thereof, such as the C₁-C₄-acyl derivatives thereof such as N-acetylcysteamine and N-propionylcysteamine, thiolactic acid and esters thereof, such as glycerol monothiolactate, thioglycolic acid and esters thereof, such as glycerol or glycol monothioglycolate, and thioglycerol.

7. Method according to any one of the preceding claims, **characterized in that** the thiols are used in the form of salts.

8. Method according to any one of the preceding claims, **characterized in that** the reducing composition is in the form of a thickened or non-thickened lotion, a cream, a gel or a mousse.

9. Method according to any one of the preceding claims, **characterized in that** the temperature is increased up to a temperature of between 60°C and 250°C, preferably between 120°C and 220°C.

10. Method according to any one of the preceding claims, **characterized in that** the reducing composition is applied to clean wet hair fibres.

11. Method according to any one of the preceding claims, **characterized in that**, after applying the reducing composition, said composition is left on.

12. Method according to any one of the preceding claims, **characterized in that** the hair fibres are not rinsed before the temperature increasing step.

13. Method according to any one of the preceding claims, **characterized in that** it comprises a step of partially predrying the hair fibres before the temperature increasing step.

14. Use of the method according to any one of the preceding claims, for modifying in a long-lasting manner the shape of the head of hair with a low hair colour degradation and/or with a low fibre degradation.
